# EUROPÄISCHE PATENTSCHRIFT

(11) **EP 1 019 118 B1**
(45) Veröffentlichungstag und Bekanntmachung des Hinweises auf die Patenterteilung: **26.11.2003**
(21) Anmeldenummer: 98949987.6
(22) Anmeldetag: 03.09.1998
(51) Int. Cl.: A61M 1/14, A61M 1/34, A61M 1/36, A61M 5/168

(54) **VERFAHREN UND VORRICHTUNG ZUR ÜBERWACHUNG EINES GEFÄSSZUGANGES WÄHREND EINER EXTRAKORPORALEN BLUTBEHANDLUNG**
METHOD AND DEVICE FOR MONITORING VESSEL ACCESS DURING EXTRACORPOREAL BLOOD TREATMENT
PROCEDE DE SURVEILLANCE DE L'ACCES A UN VAISSEAU PENDANT UN TRAITEMENT SANGUIN EXTRACORPOREL

(30) Priorität: 06.09.1997 DE 19739099
(43) Veröffentlichungstag der Anmeldung: 19.07.2000
(73) Patentinhaber: Fresenius Medical Care Deutschland GmbH, 61352 Bad Homburg v.d.H. (DE)
(72) Erfinder: ENDER, Helmuth, D-97479 Zeil (DE)
(74) Vertreter: Luderschmidt, Schüler & Partner GbR
(86) Internationale Anmeldenummer: EP9805599
(87) Internationale Veröffentlichungsnummer: WO99012588

(56) Entgegenhaltungen:
- EP-A- 0 745 400
- WO-A-97/10013

## Beschreibung

Die Erfindung betrifft ein Verfahren zur Überwachung eines Gefäßzuganges während einer extrakorporalen Blutbehandlung, insbesondere einer Dialysebehandlung oder einer Blutseparation und eine Vorrichtung zur extrakorporalen Blutbehandlung, insbesondere einer Dialysebehandlung oder einer Blutseparation, mit einer Einrichtung zur Überwachung eines Gefäßzuganges.

Zur Entfernung von harnpflichtigen Substanzen wird das Blut eines Patienten in einem extrakorporalen Blutkreislauf durch die Kammer eines von einer semipermeablen Membran in zwei Kammern unterteilten Dialysators geleitet, während die andere Kammer von einer Dialysierflüssigkeit durchströmt wird. Als Zugang zum Gefäßsystem wird häufig eine arteriovenöse Fistel angelegt, es ist aber auch der Einsatz eines Implantats möglich. Das Blut wird dem Patienten über eine arterielle Nadel entnommen, die an die arterielle Blutleitung des extrakorporalen Blutkreislaufs angeschlossen ist und wird dem Patienten über eine venöse Nadel, die an die venöse Blutleitung angeschlossen ist, wieder zugeführt.

Zur Gewinnung von Blutkomponenten, die zur Behandlung von Patienten mit bestimmten Krankheiten benötigt werden, finden Zellseparatoren Verwendung, in denen das Blut eines Spenders in einem extrakorporalen Kreislauf einer Dichtezentrifugation unterworfen und in seine Bestandteile getrennt wird.

Für die Sicherheit des Patienten während einer extrakorporalen Blutbehandlung, beispielsweise einer Dialysebehandlung oder einer Blutseparation, ist eine Überwachung des Gefäßzuganges von entscheidender Bedeutung. So ist das Herausrutschen der venösen Nadel beispielsweise mit einem größeren Blutverlust für den Patienten verbunden, wenn dieser Fehler nicht sofort bemerkt wird.

Aus dem Bereich der Infusionstechnik sind Schutzsysteme zur Überwachung des Gefäßzuganges bekannt. Die EP-A-0 328 162 beschreibt eine Infusionsvorrichtung, die einen Druckwandler in der Infusionsleitung aufweist, mit dem sich die Herzschläge des Patienten nachweisen lassen, sofern die Nadel zu dem Gefäßsystem einen Zugang hat. Ein fehlerhafter Gefäßzugang wird dadurch erkannt, daß die Herzschläge nicht mehr als Druckpulse in der Infusionsleitung gemessen werden.

Aus der EP-A-0 328 163 ist eine Infusionsvorrichtung bekannt, bei der die von der Infusionspumpe in der Infusionsleitung erzeugten Druckpulse überwacht werden. Das Herausrutschen einer Nadel wird durch eine Veränderung der Form der Druckpulse erkannt.

Derartige Schutzsysteme werden auch in Dialysevorrichtungen eingesetzt. Eine bekannte Dialysevorrichtung mit einer Einrichtung zur Überwachung des Gefäßzuganges weist einen in der venösen Blutleitung angeordneten Druckwandler auf. Mit dem Druckwandler wird ein Druckabfall erkannt, der beim Herausrutschen der Nadel auftritt. Eine Studie über die venöse Drucküberwachung bei Dialysevorrichtungen hat aber gezeigt, daß die Überwachung des venösen Rücklaufdrucks als Schutzsystem gegenüber Blutverlust in die Umgebung bei herausgerutschter Nadel versagen kann.

Die WO97/10013 beschreibt eine Dialysevorrichtung mit einem Überwachungssystem, das die in der arteriellen Blutleitung durch die Blutpumpe erzeugten Druckpulse in der venösen Blutleitung überwacht.

Aus der EP-A-0 745 400 ist eine Vorrichtung zur Überwachung der Okklusionsstellung einer peristaltischen Pumpe bekannt, in die eine Schlauchleitung eingelegt ist. Bei der bekannten Vorrichtung wird über einen Shunt eine Leiterschleife geschaffen. Der Shunt stellt eine elektrische Verbindung zwischen dem Abschnitt der Schlauchleitung stromauf der Pumpenrollen und dem Abschnitt stromab der Pumpenrollen her. Zur Überwachung der Okklusionsstellung wird mit einer stromauf der Pumpenrollen an der Schlauchleitung angeordneten Erregerspule ein elektrischer Strom in der Leiterschleife induziert, der mit einer stromab der Pumpenrolle angeordneten Induktionsspule überwacht wird. Dabei wird von der Stärke des in der Leiterschleife fließenden Stromes auf die Okklusionsstellung der peristaltischen Pumpe geschlossen.

Der Erfindung liegt die Aufgabe zugrunde, ein Verfahren zur Überwachung eines Gefäßzuganges während einer extrakorporalen Blutbehandlung zu schaffen, das die Erkennung eines fehlerhaften Gefäßzuganges mit hoher Zuverlässigkeit erlaubt, ohne daß umfangreiche Veränderungen an der Blutbehandlungsvorrichtung erforderlich sind. Diese Aufgabe wird erfindungsgemäß mit den Merkmalen des Patentanspruchs 1 gelöst.

Eine weitere Aufgabe der Erfindung liegt darin, eine Vorrichtung zur extrakorporalen Blutbehandlung mit einer Einrichtung zur Überwachung eines Gefäßzuganges zu schaffen, die einen fehlerhaften Gefäßzugang mit hoher Zuverlässigkeit erkennt und mit verhältnismäßig einfachen technischen Mitteln realisiert werden kann. Die Lösung dieser Aufgabe erfolgt erfindungsgemäß mit den Merkmalen des Patentanspruchs 6.

Es hat sich überraschenderweise gezeigt, daß der extrakorporale Blutkreislauf, der den arteriellen Gefäßzugang, die arterielle Blutleitung, die Blutbehandlungseinrichtung, die venöse Blutleitung, den venösen Gefäßzugang und das verbindende Gefäßsystem des Patienten oder Spenders umfaßt, eine geschlossene Leiterschleife bildet, in der ein elektrischer Strom fließen kann. Messungen haben ergeben, daß der Stromkreis weder durch die in den extrakorporalen Kreislauf geschaltete Blutpumpe, die auch als okkludierende Rollenpumpe ausgebildet sein kann, noch durch eine in die venöse Blutleitung geschaltete Tropfkammer ausreichend unterbrochen wird.

Bei dem erfindungsgemäßen Verfahren bzw. der erfindungsgemäßen Vorrichtung wird ein elektrischer Stromfluß in der geschlossenen Leiterschleife bewirkt, wobei der in der Leiterschleife fließende Strom gemessen und bei einer charakteristischen Veränderung der Stromstärke auf einen fehlerhaften Gefäßzugang geschlossen wird. Ein fehlerhafter Gefäßzugang liegt dann vor, wenn der Stromkreis. unterbrochen ist, d.h. die venöse bzw. arterielle Nadel der Blutleitung herausgerutscht ist.

Die Impedanzmessung erfolgt vorzugsweise mit einem Wechselstrom, dessen Amplitude und Frequenz so zu wählen ist, das einerseits eine Gefährdung des Patienten oder Spenders sowie eine Schädigung des Blutes nicht auftreten kann und andererseits eine Signalauswertung mit hoher Zuverlässigkeit möglich ist.

Das erfindungsgemäße Verfahren bzw. die erfindungsgemäße Vorrichtung zur Überwachung eines Gefäßzuganges kann in vorteilhafter Weise bei allen Blutbehandlungsverfahren Verwendung finden, die von einem extrakorporalen Blutkreislauf Gebrauch machen. Zu diesen zählen beispielsweise Dialyseverfahren oder Verfahren zur Separation des Blutes in einzelne Blutbestandteile. Unter einer Blutbehandlungseinrichtung als Bestandteil einer Vorrichtung zur Blutbehandlung wird beispielsweise ein Dialysator, Filter oder eine Separationseinheit eines Zellseparators verstanden.

Um einen Eingriff in das bestehende Schlauchsystem einer Blutbehandlungsvorrichtung zu vermeiden, wird der Strom zweckmäßigerweise induktiv in die Leiterschleife eingekoppelt. Prinzipiell können in die Blutleitungen aber auch elektrische Kontakte integriert sein.

Vorzugsweise erfolgt die induktive Einkopplung mittels einer von Wechselstrom durchflossenen Erregerspule, die an einer ersten Stelle des extrakorporalen Blutkreislaufs angeordnet ist und die induktive Auskopplung der Meßsignale mit einer Induktionsspule, die an einer zweiten Stelle des extrakorporalen Blutkreislaufs angeordnet ist. Wichtig für die Induktion einer elektromotorischen Kraft längs der Leiterschleife ist ein sich zeitlich änderndes, über die von der Leiterschleife eingeschlossene Fläche integriertes Magnetfeld.

Die Erreger- und Induktionsspulen können grundsätzlich an jeder Stelle des extrakorporalen Blutkreislaufs angeordnet sein, entscheidend ist aber, daß die Induktionsspule nicht im Streufeld der Erregerspule liegt. Vorzugsweise sind die Erreger- bzw. Induktionsspulen an der arteriellen bzw. venösen Blutleitung angeordnet.

Für den Fall, daß ein fehlerhafter Gefäßzugang erkannt wird, wird vorzugsweise ein Alarm gegeben. Darüber hinaus kann der Blutfluß im extrakorporalen Kreislauf unterbrochen werden, um einen Blutverlust zu vermeiden. Die Unterbrechung des Blutflusses kann mit einem im extrakorporalen Kreislauf angeordneten Sperrorgan, z.B. einer Schlauchklemme, erfolgen.

Das erfindungsgemäße Verfahren, kann auch mit anderen Verfahren zur Erkennung eines fehlerhaften Gefäßzuganges, beispielsweise der Überwachung eines Druckabfalls im extrakorporalen Kreislauf, kombiniert werden. Dadurch wird die Sicherheit des Überwachungssystems noch weiter erhöht.

Mit dem erfindungsgemäßen Verfahren bzw. der erfindungsgemäßen Vorrichtung kann nicht nur der Gefäßzugang überwacht werden, sondern es ist auch eine gleichzeitige Überwachung der in der venösen Leitung der bekannten Blutbehandlungsvorrichtungen angeordneten Absperrklemme möglich.

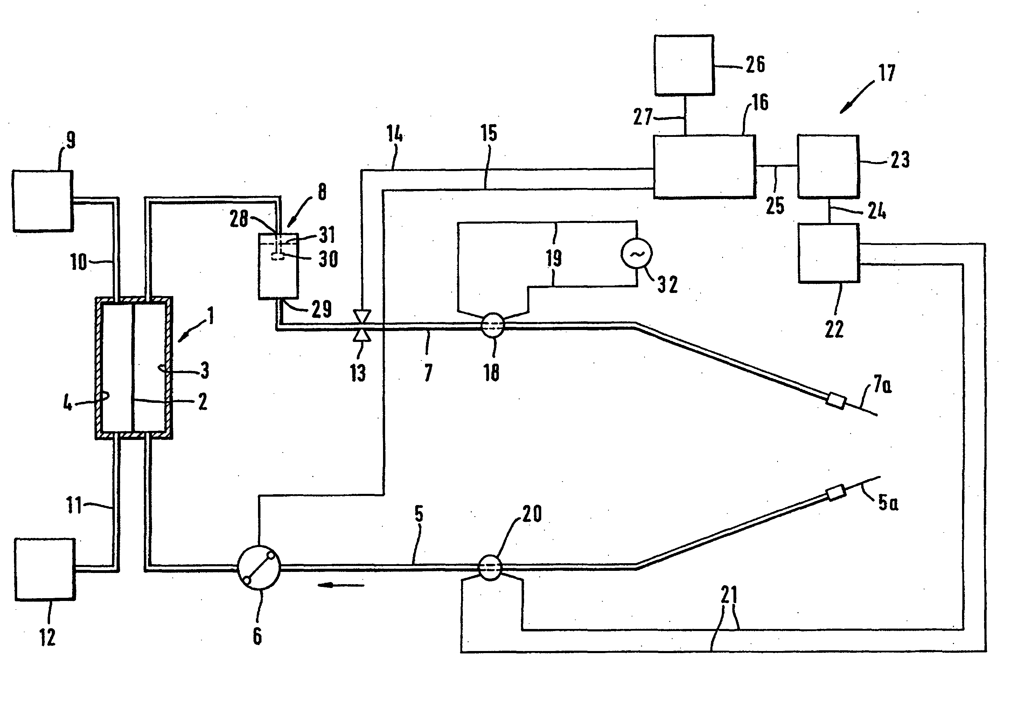

Im folgenden wird ein Ausführungsbeispiel der Erfindung unter Bezugnahme auf die Zeichnung näher erläutert.

Die Zeichnung zeigt eine Dialysevorrichtung mit einer Einrichtung zur Überwachung eines Gefäßzuganges in vereinfachter schematischer Darstellung.

Die Dialysevorrichtung weist als Blutbehandlungseinrichtung einen Dialysator 1 auf, der durch eine semipermeable Membran 2 in eine Blutkammer 3 und eine Dialysierflüssigkeitskammer 4 unterteilt ist. An dem Einlaß der Blutkammer ist eine arterielle Blutleitung 5 angeschlossen, in die eine peristaltische Blutpumpe 6 geschaltet ist. Stromab der Blutkammer 3 führt eine venöse Blutleitung 7 von dem Auslaß der Blutkammer zu dem Patienten. In die venöse Blutleitung 7 ist eine Vorrichtung zum Abscheiden von Luftblasen 8 geschaltet. An den Enden der arteriellen und venösen Blutleitung 5, 7 sind Nadeln 5a, 7a angeschlossen, die in den Patienten gestochen werden. Die arterielle und venöse Blutleitung sind Bestandteil eines als Disposable ausgebildeten Schlauchleitungssystems.

In einer Dialysierflüssigkeitsquelle 9 wird frische Dialysierflüssigkeit bereitgestellt. Von der Dialysierflüssigkeitsquelle 9 führt eine Dialysierflüssigkeitszuführleitung 10 zu dem Eingang der Dialysierflüssigkeitskammer 4 des Dialysators 1, während eine Dialysierflüssigkeitsabführleitung 11 von dem Ausgang der Dialysierflüssigkeitskammer zu einem Abfluß 12 führt.

Die Dialysevorrichtung kann noch über weitere Komponenten, z.B. eine Bilanziereinrichtung etc., verfügen, die der besseren Übersichtlichkeit halber nicht dargestellt sind.

Zur Unterbrechung des Blutflusses ist an der venösen Blutleitung 7 stromab der Vorrichtung zum Abscheiden von Luftblasen 8 eine Absperrklemme 13 vorgesehen, die elektromagnetisch betätigt wird. Die arterielle Blutpumpe 6 und die venöse Absperrklemme 13 werden über Steuerleitungen 14, 15 von einer Steuereinheit 16 angesteuert.

Die Einrichtung 17 zur Überwachung eines Gefäßzuganges weist eine Erregerspule 18 auf, die über elektrische Verbindungsleitungen 19 an einen Wechselspannungsgenerator 32 angeschlossen ist. Die Erregerspule 18 ist an einer Stelle der venösen Blutleitung 7 stromab der Schlauchklemme 13 derart angeordnet, daß deren Magnetfeld die von der Leiterschleife eingeschlossene Fläche durchsetzt. Vorzugsweise ist die Erregerspule 18 eine Zylinderspule, deren Längsachse senkrecht auf der Leiterschleifenfläche steht. Dabei kann die venöse Blutleitung 7 entweder durch die Erregerspule verlaufen oder die Erregerspule kann neben der Blutleitung liegen, wobei die Längsachse der Erregerspule mit der Blutleitung vorzugsweise einen rechten Winkel einschließt. Die Überwachungseinrichtung 17 umfaßt darüber hinaus eine Induktionsspule 20, die an der arteriellen Blutleitung 5 an einer Stelle stromauf der Blutpumpe 6 angeordnet ist. Über elektrische Verbindungsleitungen 21 ist die Induktionsspule 20 an einem Meßverstärker 22 angeschlossen. Die Induktionsspule 20 ist ebenfalls eine Zylinderspule, deren Längsachse senkrecht auf der Leiterschleifenfläche steht, wobei die arterielle Blutleitung entweder durch die Induktionsspule 20 verlaufen oder die Induktionsspule 20 neben der arteriellen Blutleitung 5 angeordnet sein kann.

Während das Blut des Patienten in einem geschlossenen Kreislauf, der eine elektrische Leiterschleife darstellt, durch die arterielle Blutleitung 5 mit dem in die Blutpumpe 6 eingelegten Schlauchabschnitt, die Blutkammer 3 des Dialysators 1, die Vorrichtung zum Abscheiden von Luftblasen 8 und die venöse Blutleitung 7 sowie das Gefäßsystem strömt, induziert die Erregerspule 18 einen Wechselstrom im extrakorporalen Blutkreislauf, dessen Stärke von der Leitfähigkeit des Blutes, dem Schlauchquerschnitt etc. abhängig ist. Der in der Leiterschleife fließende Wechselstrom erzeugt wiederum ein sich zeitlich änderndes Magnetfeld, das in der Induktionsspule 20 an der arteriellen Blutleitung eine Spannung induziert, deren Höhe von der Stärke des in der Leiterschleife fließenden Stromes abhängig ist. Wenn eine der beiden Nadeln 5a, 7a der arteriellen und venösen Blutleitung 5, 7 herausrutscht, ist der Stromkreis unterbrochen, so daß in der Induktionsspule 20 keine Spannung induziert wird.

Zur Überwachung des Gefäßzuganges wird das Ausgangssignel des Meßverstärkers 22 in einem Komparator 23, der über eine Signalleitung 24 mit dem Ausgang des Meßverstärkers 22 verbunden ist, mit einer vorgegebenen Referenzspannung verglichen. Fällt das Ausgangssignal des Meßverstärkers 22 unter die Referenzspannung ab, so erzeugt der Komparator 23 ein Steuersignal, daß die Steuereinheit 16 über eine Signalleitung 25 empfängt. Die Steuereinheit 16 schaltet dann die Blutpumpe 6 ab und betätigt die Absperrklemme 13 in der venösen Blutleitung 7, um einen Blutverlust zu verhindern. Ferner erzeugt die Steuereinheit 16 ein Alarmsignal, das ein Alarmgeber 26 über eine Signalleitung 27 empfängt, der einen akustischen und/oder optischen Alarm gibt.

Um eine geschlossene Leiterschleife zu schaffen, ist die Vorrichtung zum Abscheiden von Luftblasen 8 vorzugsweise derart ausgebildet, daß diese von dem Blut kontinuierlich durchströmt wird. Eine derartige Vorrichtung zum Abscheiden von Luftblasen ist beispielsweise in der DE 195 06 506 A1 beschrieben, auf die ausdrücklich Bezug genommen wird. Die Vorrichtung zum Abscheiden von Blut weist eine im wesentlichen kreiszylinderförmige Kammer und einen in Längsrichtung der Kammer angeordneten Einlauf- und Auslaufstutzen 28, 29 auf. An dem Einlaufstutzen 28 ist ein Strömungsleitbauteil 30 mit einem in Längsrichtung der Kammer verlaufenden zentralen Strömungsrohr befestigt, das in zwei Strömungsleitrohre übergeht. Der Stromkreis wird durch die Vorrichtung zum Abscheiden von Luftblasen nicht unterbrochen, da das Strömungsleitbauteil unterhalb des Flüssigkeitsspiegels 31 in der Kammer liegt.

## Patentansprüche

1. Verfahren zur Überwachung eines Gefäßzuganges während einer extrakorporalen Blutbehandlung, bei der einem Patienten oder Spender entnommenes Blut durch eine arterielle Blutleitung eines extrakorporalen Blutkreislaufs in eine Blutbehandlungseinrichtung strömt und aus der Blutbehandlungseinrichtung durch eine venöse Blutleitung des extrakorporalen Blutkreislaufs zurück in den Patienten oder Spender strömt,
**dadurch gekennzeichnet,**
**daß** ein elektrischer Stromfluß in der eine geschlossene Leiterschleife darstellenden Verbindung des extrakorporalen Blutkreislaufs und des Gefäßsystems des Patienten oder Spenders bewirkt wird und der in der Leiterschleife fließende Strom gemessen wird und daß bei einer charakteristischen Veränderung der Stromstärke auf einen fehlerhaften Gefäßzugang geschlossen wird.

2. Verfahren nach Anspruch 1, **dadurch gekennzeichnet, daß** eine von einem Wechselstrom durchflossene Erregerspule an einer ersten Stelle des extrakorporalen Blutkreislaufs angeordnet wird und daß eine Induktionsspule an einer zweiten Stelle des extrakorporalen Blutkreislaufs, die von der ersten Stelle verschieden ist, angeordnet und der in der Induktionsspule induzierte Strom zur Erkennung des fehlerhaften Gefäßzuganges überwacht wird.

3. Verfahren nach Anspruch 2, **dadurch gekennzeichnet, daß** die erste Stelle des extrakorporalen Blutkreislaufs an der arteriellen bzw. venösen Blutleitung und die zweite Stelle des extrakorporalen Blutkreislaufs an der venösen bzw. arteriellen Blutleitung liegt.

4. Verfahren nach einem der Ansprüche 1 bis 3, **dadurch gekennzeichnet, daß** bei Feststellung eines fehlerhaften Gefäßzuganges ein Alarm ausgelöst wird.

5. Verfahren nach einem der Ansprüche 1 bis 4, **dadurch gekennzeichnet, daß** bei Feststellung eines fehlerhaften Gefäßzuganges der Blutfluß im extrakorporalen Kreislauf unterbrochen wird.

6. Vorrichtung zur extrakorporalen Blutbehandlung mit
einer arteriellen Blutleitung (5) eines extrakorporalen Blutkreislaufs zum Anschluß an einen Patienten oder Spender, die an einem Ende mit dem Einlaß einer Blutbehandlungseinrichtung (1) verbunden ist und an dem anderen Ende mit dem Gefäßsystem des Patienten oder Spenders verbindbar ist,
einer venösen Blutleitung (7) des extrakorporalen Blutkreislaufs zum Anschluß an den Patienten oder Spender, die an einem Ende mit dem Auslaß der Blutbehandlungseinrichtung (1) verbunden ist und an dem anderen Ende mit dem Gefäßsystem des Patienten oder Spenders verbindbar ist, und
einer Einrichtung (17) zur Überwachung eines Gefäßzuganges,
**dadurch gekennzeichnet,**
**daß** an einer ersten Stelle des extrakorporalen Blutkreislaufs Mittel (18) zum Bewirken eines elektrischen Stromflusses in der eine geschlossene Leiterschleife darstellenden Verbindung des extrakorporalen Kreislaufs und des Gefäßsystems des Patienten oder Spenders und an einer zweiten Stelle des extrakorporalen Blutkreislaufs, die von der ersten Stelle verschieden ist, Mittel (20) zum Messen des in der Leiterschleife fließenden Stromes vorgesehen sind und daß eine Auswerteeinheit (22, 23) vorgesehen ist, die bei einer charakteristischen Veränderung der Stromstärke einen fehlerhaften Gefäßzugang erkennt.

7. Vorrichtung nach Anspruch 6, **dadurch gekennzeichnet, daß** ein Wechselspannungsgenerator (32) vorgesehen ist, der an einer Erregerspule (18) angeschlossen ist, die an der ersten Stelle des extrakorporalen Blutkreislaufs angeordnet ist und daß eine Induktionsspule (20) an der zweiten Stelle des extrakorporalen Blutkreislaufs angeordnet ist.

8. Vorrichtung nach Anspruch 7, **dadurch gekennzeichnet, daß** die Auswerteeinheit (22, 23) einen Komparator (23) aufweist, der die in der Induktionsspule (20) induzierte Spannung mit einem vorgegebenen Schwellwert vergleicht, wobei die Auswerteeinheit derart ausgebildet ist, daß diese einen fehlerhaften Gefäßzugang erkennt, wenn die gemessene Spannung kleiner als der Schwellwert ist.

9. Vorrichtung nach einem der Ansprüche 6 bis 8, **dadurch gekennzeichnet, daß** die Mittel (18) zum Induzieren eines Stromes und die Mittel (20) zum Messen des induzierten Stromes an der arteriellen Blutleitung (5) bzw. der venösen Blutleitung (7) angeordnet sind.

10. Vorrichtung nach einem der Ansprüche 6 bis 9, **dadurch gekennzeichnet, daß** ein Alarmgeber (26) vorgesehen ist, der bei Feststellung eines fehlerhaften Gefäßzuganges einen Alarm auslöst.

11. Vorrichtung nach einem der Ansprüche 6 bis 10, **dadurch gekennzeichnet, daß** Mittel (16, 13, 6) zum Unterbrechen des Blutflusses im extrakorporalen Blutkreislauf bei der Feststellung eines fehlerhaften Gefäßzuganges vorgesehen sind.

## Claims

1. Procedure for monitoring free access to a blood vessel during an extra-corporeal blood-treatment where blood taken from a patient or a donor flows through an arterial blood conveying tube of an extra-corporeal blood circuit into a blood treatment device and leaves the blood treatment device through a venous blood conveying tube of the extra-corporeal blood circuit to pass back into the patient or the donor, **characterised in that**, an electrical current is generated in the connected extra-corporeal blood circuit and the blood-vessel system of the patient or donor, which taken together have the nature of a closed conductive loop, and the strength of the electrical current flowing in the conductive loop is measured and that a characteristic change in the strength of the electrical current indicates that free access to a blood vessel is impeded.

2. Procedure in accordance with claim 1, **characterised in that** an excitation coil supplied with alternating current is located at a first point in the extra-corporeal blood circuit and that an induction coil is located at a second point in the extra-corporeal blood circuit - which is different to that of the first point - and the electric current induced in the induction coil is monitored to ensure detection when free access to a blood vessel is impeded.

3. Procedure in accordance with claim 2, **characterised in that** the first point in the extra-corporeal blood circuit is located in the arterial or venous blood conveying tube and the second point is located in the venous or arterial blood conveying tube.

4. Procedure in accordance with one of claims 1 to 3, **characterised in that** an alarm is given if it is established that access to a blood vessel is impeded.

5. Procedure in accordance with one of the claims 1 to 4, **characterised in that** the blood flow through the extra-corporeal circuit is interrupted if it is established that access to a blood vessel is impeded.

6. Device for extra-corporeal blood treatment with
an arterial blood conveying tube (5) of an extra-corporeal blood circuit for connecting to a patient or a donor which at one end is connected to the inlet to a blood treatment device (1) and at the other end is capable of being connected to the blood vessel system of the patient or the donor,
a venous blood-conveying tube (7) of the extra-corporeal blood circulation system for connecting to the patient or donor which at one end is connected to the outlet of the blood treatment device (1) and at the other end is capable of being connected to the blood vessel system of the patient or the donor, and
a device (17) for monitoring access to a blood vessel
**characterised in that**
at a first point in the extra-corporeal blood circuit a means (18) is provided to generate a flow of electrical current in the connected extra-corporeal circuit and the blood vessel system of the patient or donor, which taken together have the nature of a closed conductive loop, and that at a second point in the extra-corporeal blood circuit - which is different to that of the first point - a means (20) is provided to measure the strength ofthe electrical current flowing in the closed conductive loop and that an evaluation unit (22, 23) is provided which indicates that access to a blood vessel is impeded when there is a characteristic change in the strength of the electric current.

7. Device in accordance with claim 6, **characterised in that** an alternating-voltage generator (32) is provided which is connected to an excitation coil (18) which is located at the first point of the extra-corporeal blood circuit and that an induction coil (20) is located at the second point of the extra-corporeal blood circuit.

8. Device in accordance with claim 7, **characterised in that** the evaluation unit (22, 23) is provided with a comparator (23) which compares the voltage induced in the induction coil (20) with a pre-selected threshold level whereby the evaluation unit is designed in such a manner that if the measured voltage is less than the threshold value determines that access to a blood vessel is impeded.

9. Device in accordance with one of claims 6 to 8, **characterised in that** the means (18) of inducing an electrical current and the means (20) of measuring the induced current are located in the arterial blood conveying tube (5) or the venous blood conveying tube (7).

10. Device in accordance with one of the claims 6 to 9, **characterised in that** an alarm device (26) is provided which gives an alarm signal if it is established that access to a blood vessel is impeded.

11. Device in accordance with one of the claims 6 to 10, **characterised in that** means (16, 13, 6) are provided to interrupt the blood flow in the extra-corporeal blood circuit if it is established that access to a blood vessel is impeded.

## Revendications

1. Procédé pour la surveillance d'un accès aux vaisseaux au cours d'un traitement de sang extracorporel, dans lequel le sang prélevé au patient ou au donneur s'écoule dans une conduite de sang artériel d'une circulation de sang extracorporelle dans un dispositif de traitement de sang et s'écoule à partir du dispositif de traitement de sang à travers une conduite de sang veineux de la circulation de sang extracorporelle en retournant au patient ou au donneur.
**caractérisé**
**en ce qu'**on induit un courant électrique dans l'assemblage qui représente une boucle conductrice fermée de la circulation de sang extracorporelle et du système vasculaire du patient ou du donneur, et on mesure le courant qui passe dans la boucle conductrice et en ce que pour un changement caractéristique de l'intensité de courant l'accès défectueux aux vaisseaux est fermé.

2. Procédé selon la revendication 1, **caractérisé en ce qu'**une bobine d'excitation à travers laquelle passe un courant alternatif est disposée à un premier point de la circulation de sang extracorporelle et **en ce qu'**une bobine d'induction est disposée à un second point de la circulation de sang extracorporelle, qui est différent du premier point, et on surveille le courant induit par la bobine d'induction pour déceler l'accès défectueux au vaisseau.

3. Procédé selon la revendication 2, **caractérisé en ce que** le premier point de la circulation de sang extracorporelle est situé sur la conduite de sang artériel ou veineux et le second point de la circulation de sang extracorporelle est situé sur la conduite de sang veineux ou artériel.

4. Procédé selon l'une des revendications 1 à 3 , **caractérisé en ce que** la détection d' un accès défectueux au vaisseau déclenche une alarme.

5. Procédé selon l'une des revendications 1 à 4, **caractérisé en ce que**, lorsqu'on détecte un accès défectueux au vaisseau, le flux de sang est interrompu dans la circulation de sang extracorporelle.

6. Dispositif pour le traitement de sang extracorporel présentant
une conduite de sang artériel (5) d'une circulation de sang extracorporelle pour raccorder à un patient ou donneur, dont une extrémité est reliée à l'orifice d'entrée d'un dispositif de traitement de sang (1) et l'autre extrémité au système vasculaire du patient ou du donneur,
une conduite de sang veineux (7) de la circulation de sang extracorporelle à raccorder au patient ou au donner, dont une extrémité est reliée à l'orifice d'évacuation du système de traitement de sang extracorporel (1) et l'autre extrémité au système vasculaire du patient ou donneur, et
un dispositif (17) pour la surveillance d'un accès au vaisseau,
**caractérisé**
**en ce que**, à un premier point de la circulation de sang extracorporelle, on prévoit des moyens (18) pour induire un courant électrique dans l'assemblage représentant une boucle conductrice fermée de la circulation de sang extracorporelle et le système vasculaire du patient ou du donneur, et à un second point de la circulation de sang extracorporelle, qui est différents du premier point, on prévoit des moyens (20) pour mesurer le courant qui passe par la boucle conductrice et en ce qu'on prévoit une unité d'évaluation (22, 23), qui reconnaît un accès défectueux au vaisseau lors d'un changement caractéristique de l'intensité de courant.

7. Dispositif selon la revendication 6, **caractérisé en ce qu'**on prévoit un générateur de courant alternatif (32), qui est relié à une bobine d'excitation (18), qui est disposé au premier point de la circulation de sang extracorporelle et **en ce qu'**une bobine d'induction (20) est disposée au second point de la circulation de sang extracorporelle.

8. Dispositif selon la revendication 7, **caractérisé en ce que** l'unité d'évaluation (22, 23) présente un comparateur (23), qui compare la tension induite par la bobine inductrice (20) avec la valeur de seuil fixée auparavant, l'unité d'évaluation étant conçue de manière telle qu'elle reconnaisse un accès défectueux au vaisseau lorsque la tension mesurée est inférieure à la valeur de seuil.

9. Dispositif selon la revendication 6 à 8, **caractérisé en ce que** les moyens (18) pour l'induction d'un courant et les moyens (20) pour mesurer le courant induit sont disposés dans la conduite de sang artériel (5) ou la conduite de sang veineux (7).

10. Dispositif selon l'une des revendications 6 à 9, **caractérisé en ce qu'**il est prévu un dispositif d'alarme (26), qui déclenche une alarme lors de la détection d'un accès défectueux au vaisseau.

11. Dispositif selon l'une des revendications 6 à 10, **caractérisé en ce que** ce qu'on prévoit des moyens (16, 13, 6) pour l' interruption du flux de sang dans la circulation de sang extracorporelle lors de la détection d'un accès défectueux au vaisseau.
